# EUROPEAN PATENT APPLICATION

(11) **EP 1 964 558 A1**
(43) Date of publication of application: **03.09.2008**
(21) Application number: 06783801.1
(22) Date of filing: 12.07.2006
(51) Int. Cl.: A61K 31/365, A61K 31/455, A61P 3/04

(54) **PHARMACEUTICAL COMPOSITIONS CONTAINING INTESTINAL LIPASE INHIBITING SUBSTANCES COMBINED WITH A CHROMIUM DINICOTINATE O-COORDINATED COMPLEX FOR USE IN THE TREATMENT AND CONTROL OF OBESITY AND OVERWEIGHT**

(30) Priority: 20.12.2005 MX PA05014090
(71) Applicant: Espinosa Abdala, Leopoldo de Jesús, C.P. 45110 Zapopan, Jalisco , México (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco (MX); ÁLVAREZ OCHOA, Victor Guillermo, C.P. 45222 Zapopan, Jalisco, México (MX); SANTOS MURILLO, Josefina, C.P. 44600 Zapopan, Jalisco, México (MX); GARCÍA ARMENTA, Patricia, C.P. 45110 Zapopan, Jalisco, México (MX); ESPINOSA ABDALA, Leopoldo de Jesus, C.P. 45110 Zapopan, Jalisco (MX)
(74) Representative: Carvajal y Urquijo, Isabel
(86) International application number: PCT/MX2006/000071
(87) International publication number: WO 2007/073134

(57) **Abstract**

The invention relates to the pharmaceutical industry in general and to the pharmaceutical industry involved in the production of medicaments for controlling obesity and overweight. More specifically, the invention relates to a composition containing an intestinal lipase inhibiting substance and a chromium dinicotinate O-coordinated complex. The composition is **characterized in that** the aforementioned combination or association consists of a selected intestinal lipase inhibiting substance known as Orlistat and a chromium dinicotinate O-coordinated complex, in order to product a produce which has a synergistic effect for the treatment of obesity and overweight, with fewer side effects.

## Description

### FIELD OF THE INVENTION

The present invention generally relates to pharmaceutical industry and in particular to drug preparer pharmaceutical industry for controlling obesity and overweight. More particularly, the invention relates to a combination of intestinal lipase inhibiting substances such as Orlistat combined with a chromium dinicotinate O-coordinated complex such as chromium dinicotinate.

### BACKGROUND OF THE INVENTION

In Mexico, in general, more attention has been focused on undernutrition problems that on malnutrition problems for excess. However, the changes the country has experienced and the epidemiological transition process through which it is passing indicate that malnutrition for excess can also constitute an important public health problem. The increasing urbanization and economic development produce changes in conditions and life styles. These changes can generate modifications on the diet and on the population physical activity patterns, which can increase obesity risk.

Information proceeding from both developed and developing countries indicates that the risk of suffering obesity is not homogeneous among the different socioeconomic and educational stratum of the population. In the United States, just before 1980, obesity prevalence in children was higher among high socioeconomic stratum. However, it has recently been detected a prevalence increase in low-income pre-school children. Likewise, a higher prevalence has been found among low-income children, Hispanics or American Indians, compared to that from other ethnic groups. Obesity among adults in the United States is more frequent among low-income people and among African American and Mexican American population.

The term obesity involves an excess of adipose tissue, but the complicated is to define the meaning of said excess.

When the ingestion of energy surpasses the waste of energy, the excessive calories are deposited in the adipose tissue and, if this positive balance is maintained, obesity is produced: there are therefore two components in weight balance and any anomaly of one of them (ingestion or waste) can cause obesity.

Due to its magnitude and transcendence, it is considered as a Public Health problem in Mexico, as shown by that reported by 2000 Mexican National Health Survey, that from early ages there is a high prevalence of overweight and obesity as a 29% of overweight and obesity is present in adolescents and as to adults and women ranging from 20-59 years old a 36.1% of overweight and 28.1% of obesity was present and in men from the same etarean group 40.9% of overweight and 18.6 of obesity.

**Genetic factors:** Recent researches suggest that, on average, genetic influence contributes at about a 33 percent to the body weight, but this influence can be higher or less in a particular person.

**Socioeconomic factors:** these factors strongly influence in obesity, especially in women. In some developed countries, obesity frequency is more than twice among low socioeconomic status women than among higher socioeconomic status women. The reason why socioeconomic factors have a strong influence over women's weight is not completely understood, but it is known that measures taken against obesity increase with the social status. Women who belong to groups from a higher socioeconomic status have more time and resources to be on a diet and to practice exercise which allow them adapted themselves to these social demands.

**Psychological factors:** Emotional disorders, which for some time were considered as an important cause of obesity, are currently considered as a reaction to the strong prejudices and discrimination against obese people. One of the emotional disorders type, the body negative appearance, is a serious problem for many obese young women. This leads to an extreme lack of self-confidence and discomfort in certain social situations.

**Factors relative to development:** an increase of the size or the number of adipose cells, or both, adds to the quantity of fats stored in the body. Obese people, particularly those having developed obesity during childhood, can have an amount of fat cells over five times higher than people with normal weight. As the number of cells cannot be reduced, the loss of weight can be achieved only by reducing the amount of fat on each cell.

**Physical activity:** a reduced physical activity is probably one of the main reasons for obesity increase among people from wealthy societies. In some of them, the United States, for example, obesity is, at the present time, twice more frequent than in 1900, even when the average amount of consumed calories on a daily basis has decreased a 10 percent. Sedentary people need fewer calories. The Increased physical activity makes normal-weight people to eat more, but this may not occur the same way for obese people.

**Hormones:** Some hormonal disorders such as Cushing syndrome, suprarenal insufficiency, diabetes, etc., can cause obesity.

**Brain injury:** Only in a few cases, a brain injury, especially from the hypothalamus, can result in obesity.

**Drugs:** Certain frequently used drugs such as prednisone (a corticosteroid) and many anti-depressant drugs, as well as many other drugs used to cure psychological disorders cause weight gain.

Obesity is precursor state to type-2 diabetes; however, not all obese people develop diabetes.

### OBESITY COMPLICATIONS:

Cardiovascular problems
Atherosclerosis
Type-2 diabetes mellitus
Respiratory problems
Osteoarticular problems
Digestive problems
Renal problems
Cutaneous problems
Gonadal problems
Gestational problems
Sexual problems
Neurological problems
Hematological problems
Infections
Parietal problems
Surgical and anesthetic problems
Oncologic problems
Psychological problems
Social problems
Mortality
Suicide

The number of people suffering from obesity is twice in ages from 20 and 50 years and it drastically decreases after such ages. A high frequency of chronic-degenerative diseases such as type-II diabetes mellitus is associated with people suffering from overweight and obesity, being observed in many cases that when both pathologies coexist together the glycemic control is difficult, resulting in a resistance to insulin with the catastrophic consequences of both pathologies.

There are different types of medicaments used in anti-obesity therapy; however, it has been observed that a combined therapy is more effective for this pathology compared to a monotherapy, being found high indexes of therapeutic failures.

As noted above, there are various medicaments within those used; however, is our interest to revise the intestinal lipase inhibitors and the chromium dinicotinate O-coordinated complex.

### ORLISTAT

Orlistat is a gastrointestinal lipase inhibitor produced by *Streptomyces toxytricini* which is used for treating obesity and maintaining weight in conjunction with a hypocaloric diet. Orlistat acts locally, preventing diet fats from being absorbed. Orlistat is indicated for patients with a body weight index ≥ 30 kg/m² o ≥ 27 kg/m² if other risk factors (hypertension, diabetes or dyslipidemia) are further presented. The results from a two-year duration clinical study performed in 18 centers showed a significant weight loss in patients with a body mass index between 30 and 43 kg/m². At the same time, fasting insulin and LDLs reduction was observed.

### CHROMIUM DINICOTINATE

Chromium dinicotinate O-coordinated complex. The most important natural source of chromium is brewer's yeast, chromium being also found in other food products such as red meat, liver, cheese, some nuts, germs and grain husks. Brewer's yeast contains various types of chromium-plated complexes.

Among these chromium-plated complexes only 10% presents the GTF-substrate properties and has been known as the nicotinic acid, which forms chromium-plated complexes.

These complexes have been synthesized, being chromium dinicotinate O-coordinated complex the one with most biological activity and effectiveness, being proven the insulin enhancing activity properties both in vivo and in vitro.

Chromium is a trace element which intervenes as a cofactor of various endogenous bioactive complexes; the most well-known is the niacin-chromium-niacin complex, which is an essential component of the Glucose Tolerance Factor.

This factor regulates and promotes the insulin activity by increasing the effectiveness thereof, and the energetic homeostasis is thereby maintained, providing the appropriated levels of blood glucose, which depend in turn on both pool food contribution (glucogen, fats, etc.) and requirements induced by a momentary activity of the individual.

In short, the basis on which the treatment supports are the procedures directed to unbalance the energetic balance equation in favor of the caloric consumption, especially in those patients with obesity and overweight. The pharmacological treatment is directed to prevent diet fats from being absorbed and to favor the insulin activity, through regulation and promotion, increasing the effectiveness thereof.

### BRIEF DESCRIPTION OF THE INVENTION

The present invention describes a pharmaceutical combination comprising a combination of intestinal lipase inhibiting substances such as Orlistat and/or any of the salts thereof, combined with a chromium dinicotinate O-coordinated complex such as chromium dinicotinate for pharmaceutical use of the composition for treating and controlling obesity or overweight.

After multiple tests, it could be deduced that Orlistat, in one embodiment, can be present in a concentration of 120 mg per dosage unit.

As for chromium dinicotinate, it could be proved that a suitable concentration is of 2 mg per dosage unit.

### DETAILED DESCRIPTION OF THE INVENTION

In accordance to the present invention, a pharmaceutical composition was made comprising a combination of intestinal lipase inhibiting substances with a chromium dinicotinate O-coordinated complex, which combination thereof produces a synergistic effect in weight loss, with a better activity of the insulin, with a less amount of the ingredients, being better tolerated and with fewer adverse effects in Obese patients with type-2 Diabetes.

### EXAMPLE 1

An open, prospective, double-blind comparative and crossover study was made in order to assess the percentage of moderate-severe obese patients BMI 32 to 34.9 kg/m² or with a BMI higher than 35 kg/m², with difficult control diabetes in spite of the ingestion of oral hypoglycemic agents, diet and exercise.

The study includes 36 individuals average of 48.3 years old, prevailing the male sex 22/14 compared to the female sex. The enrolled individuals had the antecedent of suffering from type-2 diabetes with an evolution time of 6 years and an irregular control in its glycemia figures, being on average of 184 mg/dl; moreover, all of the individuals had the antecedent of an regular consumption of up to 3 hypoglycemic drugs without achieving a suitable control. The study had a 24-week duration.

### Results

Once the inclusion criteria were fulfilled, the medicament under study consisting of Orlistat/chromium dinicotinate was administered to 18 individuals who constituted Group 1, in Group 2, the patients received therapy based on Orlistat alone continuing with their usual hypoglycemic scheme.

During the study in Group 1, a significant decrease in the fasting glycemic figures was observed compared to the admission, being documented figures at the basal visit from 196 mg/dl to 110 mg/dl at the end of the therapy; additionally, it was necessary to adjust the dose of oral hypoglycemic agents and to gradually reduce the number of administered drugs. An statistically significant weight loss was observed with a reduction of the BMI from 33.6kg/m² to 29.4 kg/m².

In group 2, the individuals continue presenting obesity with non-statistical reductions from about 400 to 500 g compared to the basal visit; however, an unnoticeably reduction in the glycemic levels was observed, with a better response to oral hypoglycemic agents, this report was however non-statistically significant.

An adverse event was reported characterized by semi-liquid evacuations which merit neither concomitant therapy nor to suspend the study therapy.

### Conclusions

The combination of Orlistat/chromium dinicotinate showed to be effective both in weight and BMI loss and increase of the insulin effectiveness. Resulting in a proper control of the glycemic figures in individuals suffering difficult control type-2 diabetes.

## Claims

1. Synergistic pharmaceutical composition comprising a synergistic combination of an intestinal lipase inhibitor and a chromium dinicotinate O-coordinated complex for treating obesity and overweight.

2. Pharmaceutical composition according to claim 1 comprising the synergistic combination, wherein the intestinal lipase inhibitor is Orlistat and/or any of the salts thereof and a chromium dinicotinate O-coordinated complex known as chromium dinicotinate and/or any of the salts thereof.

3. A pharmaceutical composition according to claim 2 wherein the ingredients combination provides a product with improved properties, causing a synergistic effect.

4. A pharmaceutical composition according to claim 2 wherein the ingredients combination reduces the body mass index (BMI), reduces the adipocytes (fat cells) size, increases the energetic waste by increasing heat production, without adverse events.

5. A pharmaceutical composition according to claims 2, 3, and 4, wherein the medicament requiring a fewer dose is Orlistat, for administration once a day.

6. A pharmaceutical composition according to claim 2, wherein Orlistat is present in a concentration of 120 mg per dosage unit.

7. A pharmaceutical composition according to claim 2, wherein chromium Dinicotinate is present in a concentration of 2 mg per dosage unit.

8. The use of the composition according to claim 2 for treating and controlling obesity and overweight.
